# EUROPEAN PATENT APPLICATION

(11) **EP 4 714 447 A1**
(43) Date of publication of application: **25.03.2026**
(21) Application number: 24201070.0
(22) Date of filing: 18.09.2024
(51) Int. Cl.: A61K 31/4725, A61K 31/496, A61P 35/00

(54) **DRUG COMBINATIONS OF BCL-XL INHIBITORS FOR TREATMENT OF CANCER**

(71) Applicant: Stichting Amsterdam UMC, 1081 HV Amsterdam (NL)
(72) Inventor: Medema, Jan Paul, 1081 HV Amsterdam (NL); Zhang, Le, 1081 HV Amsterdam (NL); Ramesh, Prashanti, 1081 HV Amsterdam (NL)
(74) Representative: Pot, Emil

(57) **Abstract**

The invention covers a novel combination therapy for the treatment of cancer, preferably of colorectal cancer. The inventors identified Manidipine-2HCl as a selective inhibitor of UGT8, a key enzyme in the synthesis of sulfatides. In agreement, lipidomic analysis indicated that sulfatides are strongly reduced in colorectal cancer cells upon treatment with Manidipine-2HCl. Intriguingly, this reduction led to severe mitochondrial swelling and a strong synergism with BH3 mimetics targeting BCL-XL, leading to the activation of mitochondria-dependent apoptosis. The inventors demonstrated that mechanistically, Manidipine-2HCl enhanced mitochondrial BAX localization in a sulfatide-dependent fashion, facilitating its activation by BH3 mimetics. This disclosure relates to a method of treating a patient suffering from cancer with a combination therapy comprising a BCI-XL inhibitor and a UGT8 inhibitor. This disclosure also relates to uses of such combination for preparation of a medicament for the treatment of a cancer; methods of treating a cancer in a subject in need thereof comprising administering to said subject a jointly therapeutically effective amount of said combination; pharmaceutical compositions comprising such combination and commercial packages thereto.

## Description

### Field of the invention

The invention pertains to the field of cancer in particular to methods of treating a colorectal cancer based on a combination of BCL-XL inhibitors and UGT8 inhibitors.

### Background

Colorectal cancer (CRC) is one of the most common and best studied cancers worldwide. Despite therapy innovation, many CRC patients, especially at late stage disease, still have a poor prognosis and display unresponsiveness to current therapies. Novel therapeutic modalities are therefore still urgently needed. However, during progression, cancer cells are shaped to withstand stresses, such as oncogene-induced stress, hypoxia, nutrient insufficiency and anti-cancer immune attack. All these stresses are reported to somehow trigger the apoptotic machinery, which is wired to restrain the development of cancer. Effective progression of cancer cells therefore inevitably aligns with an adaption to survive stresses and apoptotic insults, for instance through the upregulation of anti-apoptotic BCL2 family members. This adaptation endows cancer cells with apoptosis resistance. In CRC, the inventors and others have shown, that a switch towards a dependency on BCL-XL, one of the anti-apoptotic BCL2 proteins, for survival is absolutely required for progression. In agreement, CRC can be effectively targeted with BH3 mimetics that antagonize the activity of BCL-XL. BH3 mimetics act as apoptosis inducers by modulating the balance between anti-apoptotic BCL2 proteins and apoptotic executioners like BAX. When relieved of inhibition, BAX is set free to oligomerize and permeabilize the outer mitochondrial membrane (OMM), releasing cytochrome c and unleashing the downstream apoptotic machinery.

Another way cancer cells adapt to changing requirements and stress is by a complete rewiring of their metabolic pathways. Almost 100 years ago, Warburg proposed the glycolytic adaptation of cancer cells, but a much broader metabolic adaptation of multiple pathways is detected and in fact constitutes one of the hallmarks of cancer. Indeed, cancer cells acquire the capacity for *de novo* fatty acid synthesis and change their lipid metabolism to fit a growing need for fatty acids and lipids. Intriguingly, this also exposes vulnerabilities and several studies have used drugs to target lipid metabolism as a potential and promising approach in cancer treatment. Whether the metabolic adaptation is also linked to escape from stresses and apoptosis is not fully explored. However, multiple lipid species have been reported to regulate the onset and execution of apoptosis. For instance, cardiolipin is a crucial player in mitochondrial apoptosis and its constitution is adapted in cancer cells. Similarly, sphingolipids, which form a subgroup of lipids enriched in membrane micro-domains also known as lipid rafts, play a role in orchestration of signal transduction, but also regulate apoptosis. The metabolism of sphingolipids is complex and split into many connected pathways, with ceramides located centrally to many of these metabolic pathways. De novo synthesis, salvage pathways, sphingomyelin synthesis as well as glycosylation gives rise to the diversity of a sphingolipid superfamily. Although some sphingolipids have been reported to regulate mitochondrial apoptosis, their exact role remains largely unclear.

In this invention, the inventors aimed to identify sensitizers of CRC cells to low doses of the BCL-XL inhibitor, A-1155463, in order to exploit the vulnerability towards BCL-XL inhibition of these cancer cells. The inventors revealed that Manidipine-2HCl decreased the activity of UDP-glycosyltransferase 8 (UTG8) and as a result lowered sulfatide levels. Surprisingly, this strongly affected mitochondrial function, reflected by a drop in respiration, mitochondrial swelling as well as enhanced mitochondrial BAX localization, which facilitated BH3 mimetic-induced apoptosis. Our data reveal a novel regulatory level of mitochondrial-dependent apoptosis that can be exploited to kill CRC cells.

### Summary of the invention

The invention is summarized in the following embodiments:
Embodiment 1. A combination of a UGT8 inhibitor and a BCL-XL inhibitor for use in a method of treating a patient suffering from cancer.
Embodiment 2. The combination of use according to claim 1 wherein the UGT8 inhibitor is Manidipine-2HCl, RA 5557, or UGT8i19 (UGT8 inhibitor 19). More preferably the UGT8 inhibitor is Manidipine-2HCl
Embodiment 3. The combination of use according to any one of the previous claims wherein the BCL-XL inhibitor belongs to the groups of BCL-XL inhibitors such as small molecules: A-1155463, A-1331852, WEHI-539, ABT-199 (Venetoclax), ABT-263 (Navitoclax), ABT-737, TW-37, (R)-(-)-Gossypol (AT-101) acetic acid, Sabutoclax, BH3I-1, Gambogic Acid, WL-276, Obatoclax, A-793844, A-1293102; Oligomers: p-terphenyl derivative (BH3-M6), oligoamide-foldamer (JY-1-106) , naturally derived preferential or dual BCL-XL/BCL-2/MCL-1 inhibitors: Anacardic acids, Endiandric acids, Marinopyrroles, Polyphenols (Gossypol, ApoG2, EGCG, TW-37), Meiogynins, and antimycin-A. More preferably the BCL-XL inhibitor is A-1155463.
Embodiment 4. The method of treatment according to claim 1 wherein the cancer type is colorectal cancer
Embodiment 5. A pharmaceutical preparation, comprising a UGT8 inhibitor and a BCL-XL inhibitor as a combined preparation for simultaneous, separate or sequential use in the treatment of colorectal cancer in a subject.
Embodiment 6. The pharmaceutical preparation according to embodiment 5, comprising a pharmaceutical preparation comprising a UGT8 inhibitor and a pharmaceutical preparation comprising a BCL-XL inhibitor
Embodiment 7. The pharmaceutical preparation according to embodiment 5 or embodiment 6, for use in a method of treating colorectal cancer
Embodiment 8. A method of treating a colorectal cancer in a subject, the method comprising the simultaneous, separate or sequential administration to the subject of a UGT8 inhibitor and a BCL-XL inhibitor or the pharmaceutical preparation from embodiment 5 to the subject.

### Definitions

Various terms relating to the methods, compositions, formulations, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

Methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

"A," "an," and "the": these singular form terms include plural referents unless the content clearly dictates otherwise. The indefinite article "a" or "an" thus usually means "at least one". Thus, for example, reference to "a cell" includes a combination of two or more cells, and the like.

"About" and "approximately": these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods. Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified. For example, a ratio in the range of about 1 to about 200 should be understood to include the explicitly recited limits of about 1 and about 200, but also to include individual ratios such as about 2, about 3, and about 4, and sub-ranges such as about 10 to about 50, about 20 to about 100, and so forth.

"And/or": The term "and/or" refers to a situation wherein one or more of the stated cases may occur, alone or in combination with at least one of the stated cases, up to with all of the stated cases.

"Comprising": this term is construed as being inclusive and open ended, and not exclusive. Specifically, the term and variations thereof mean the specified features, steps or components are included. These terms are not to be interpreted to exclude the presence of other features, steps or components.

Exemplary": this terms means "serving as an example, instance, or illustration," and should not be construed as excluding other configurations disclosed herein.

The term "expression" may refer to the transcription and/or translation of a particular nucleotide sequence driven by a promoter.

The terms "protein" and "polypeptide" are used interchangeably and may refer to any polymer of amino acids (dipeptide or greater) linked through peptide bonds or modified peptide bonds. Polypeptides of less than about 10-20 amino acid residues are commonly referred to as "peptides." The polypeptides of the invention may comprise non-peptidic components, such as carbohydrate groups. Carbohydrates and other non-peptidic substituents may be added to a polypeptide by the cell in which the polypeptide is produced, and will vary with the type of cell. Polypeptides are defined herein, in terms of their amino acid backbone structures; substituents such as carbohydrate groups are generally not specified, but may be present nonetheless.

The terms "reduced" or "eliminated" or "disruption" or "disrupted" are used interchangeably herein to refer to any genetic modification that decreases or eliminates expression and/or the functional activity of the nucleic acid or an expression product thereof. For example, disruption of a gene includes within its scope any genetic modification that decreases or eliminates expression of the gene and/or the functional activity of a corresponding gene product ( e.g ., mRNA and/or protein). Genetic modifications include complete or partial inactivation, suppression, deletion, interruption, blockage, or down- regulation of a nucleic acid (e.g., a gene). Illustrative genetic modifications include, but are not limited to, gene knockout, inactivation, mutation (e.g., insertion, deletion, point, or frameshift mutations that disrupt the expression or activity of the gene product), or use of inhibitory nucleic acids (e.g., inhibitory RNAs such as sense or antisense RNAs, molecules that mediate RNA interference such as siRNA, shRNA, miRNA; etc.), inhibitory polypeptides (e.g., antibodies, polypeptide-binding partners, dominant negative polypeptides, enzymes etc.) or any other molecule that inhibits the activity of the LCK gene or level or functional activity of an expression product of the LCK gene.

By "pharmaceutically acceptable carrier" is meant a solid or liquid filler, diluent or encapsulating substance that can be safely used in topical or systemic administration to an animal, preferably a mammal, including humans. Representative pharmaceutically acceptable carriers include any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329, incorporated herein by reference). Except insofar as any conventional carrier is incompatible with the active ingredient(s), its use in the pharmaceutical compositions is contemplated.

The terms "cancer" and "cancerous" refer to or describe the physiological condition in mammals that is typically characterized in part by unregulated cell growth. As used herein, the term "cancer" refers to non-metastatic and metastatic cancers, including early stage and late stage cancers. The term "precancerous" refers to a condition or a growth that typically precedes or develops into a cancer. By "non-metastatic" is meant a cancer that is benign or that remains at the primary site and has not penetrated into the lymphatic or blood vessel system or to tissues other than the primary site. Generally, a non-metastatic cancer is any cancer that is a Stage 0, I, or II cancer, and occasionally a Stage III cancer. Generally, stage 0 is defined as a cancer precursor lesion, e.g., adenoma. By "early stage cancer" is meant a cancer that is not invasive or metastatic or is classified as a Stage 0, I, or II cancer. Stage III generally refers to a cancer that has started spreading into surrounding tissues and it is generally characterized by the presence of cancer cells in the lymph nodes. Stage IV generally refers to a cancer that has spread to distant organs. The term "late stage cancer" generally refers to a Stage III or Stage IV cancer, but can also refer to a Stage II cancer or a sub-stage of a Stage II cancer. One skilled in the art will appreciate that the classification of a Stage II cancer as either an early stage cancer or a late stage cancer depends on the particular type of cancer.

The term "cancer" includes but is not limited to, breast cancer, large intestinal cancer, lung cancer, small cell lung cancer, gastric (stomach) cancer, liver cancer, blood cancer, bone cancer, pancreatic cancer, skin cancer, head or neck cancer, cutaneous or intraocular melanoma, uterine sarcoma, ovarian cancer, rectal or colorectal cancer, anal cancer, colon cancer, fallopian tube carcinoma, endometrial carcinoma, cervical cancer, vulval cancer, squamous cell carcinoma, vaginal carcinoma, Hodgkin's disease, non-Hodgkin's lymphoma, esophageal cancer, small intestine cancer, endocrine cancer, thyroid cancer, parathyroid cancer, adrenal cancer, soft tissue tumor, urethral cancer, penile cancer, prostate cancer, chronic or acute leukemia, lymphocytic lymphoma, bladder cancer, kidney cancer, ureter cancer, renal cell carcinoma, renal pelvic carcinoma, CNS tumor, glioma, astrocytoma, glioblastoma multiforme, primary CNS lymphoma, bone marrow tumor, brain stem nerve gliomas, pituitary adenoma, uveal melanoma (also known as intraocular melanoma), testicular cancer, oral cancer, pharyngeal cancer or a combination thereof. Cancers that may be treated include tumors that are not vascularized, or not yet substantially vascularized, as well as vascularized tumors. The cancers may comprise non-solid tumors (such as hematological tumors, for example, leukemias and lymphomas) or may comprise solid tumors. Types of cancers to be treated with the CARs of the invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

The invention relates to methods for the treatment of cancer in patients, and in particular the treatment of bowel (colorectal) cancer. Accordingly, in some aspect the invention relates to the treatment of colon cancer in a patient. In some embodiments the colorectal cancer is microsatellite stable (MSS). That is, the tumor is categorized as MSS by genomic profiling and/or immunohistochemistry. Tumors which are not MSS are termed MSI-High (Microsatellite Instability - High) or MSI-Low. Genomic profiling to determine MSI/MSS status is described in Kawakami et al., Curr. Treat. Options Oncol., 2015 Jul;16(7):30 (PMID: 26031544) and testing systems are commercially available from, for example, Promega^{®} (OncoMate^{™} MSI Dx Analysis System). Estimates of the proportion of colorectal tumors that are MSS varies, but it is widely agreed that around 80% of colorectal tumors are MSS.

In an embodiment the cancer is colorectal cancer. The term "colorectal cancer", as used herein, refers to cancer in the colon or rectum, also known as colon cancer, rectal cancer or bowel cancer. In one embodiment, the present disclosure relates to metastatic colorectal cancer.

The combination is expected to achieve superior effects in cancer, which is characterized by one or more of KRAS mutation and/or BRAF mutation and/or MEK1 mutation and/or PIK3CA mutation and/or PIK3CA overexpression.

Patients with colorectal cancer harboring KRAS or BRAF mutations, which together make up 50%-60% of reported colorectal cancer cases (Fearon 2011), are generally associated with a poor prognosis (Arlington, Heinrich et al. 2012, Safaee Ardekani, Jafarnejad et al. 2012). The combinations of this disclosure are particularly useful for treatment of cancer, which comprises one or more of KRAS mutation or one or more of BRAF mutation.

Examples of BRAF mutations include, but not limited to V600E, R46 II, I462S, G463E, G463V, G465A, G465E, G465V, G468A, G468E, N580S, E585K, D593V, F594L, G595R, L596V, T598I, V599D, V599E, V599K, V599R, V600K, A727V. Most of these mutations are clustered to two regions: the glycine-rich P loop of the N lobe and the activation segment and flanking regions. V600E mutation has been detected in a variety of cancers, and is due to a substitution of thymine with adenine at nucleotide 1799. This leads to valine (V) being substituted for by glutamate (E) at codon 600 (now referred to as V600E). MEK1 mutation may be, for example, MEK1 S72G mutation. Examples of PIK3CA mutation and/or PIK3CA overexpression include, but not limited to , amplification of the alpha isoform of PI3K, somatic mutation of PIK3CA, germline mutations or somatic mutations of PTEN, mutations and translocation of p85ot that serve to up-regulate the p85-pl 10 complex, or amplification or overexpression of the beta isoform of PI3K.

In some embodiments, the colorectal cancer is KRAS-TP53 double mutant colon cancer. In other words, the tumor is categorized as having both the KRAS mutation and the TP53 mutation by genomic profiling.

The KRAS (Kirsten rat sarcoma virus) gene is an oncogene. KRAS mutation is thought to be associated with about 40% of colorectal cancers and may be determined by tests that are known in the field. Most methods include the use of PCR to amplify the appropriate region of the KRAS gene, including exons 2 and 3, and then utilize different methods to distinguish wild-type from mutant sequences in key codons, such as 12 and 13. The detection methods include nucleic acid sequencing, allele-specific PCR methods, single-strand conformational polymorphism analysis, melt-curve analysis, and probe hybridization. Tests for detection for KRAS mutations, such as Cobas^{®} KRAS Mutation Test (Roche) and Therascreen KRAS RGQ PCR Kit (Qiagen), are FDA approved (https://www.fda.gov/medical-devices/in-vitro-diagnostics/list- cleared-or-approved-companion-diagnostic-devices-in-vitro-and-imaging-tools).

Tumor protein p53 (TP53) is a gene that codes for a tumor suppressor protein, which regulates expression of genes involved in cell cycle arrest, apoptosis, senescence, DNA repair, and changes in metabolism. TP53 mutations are associated with Li-Fraumeni syndrome and may be determined by tests that are known in the field. Methods to detect and analyze TP53 mutations include PCR assays that look for mutations present in exons 2, 3, 4, 5, 6, 7, 8, 9,10, and 11 of TP53 (https://www.fda.gov/medical- devices/in-vitro-diagnostics/list-cleared-or-approved-companion-diagnostic-devices-in-vitro-and-imaging- tools).

The term "overexpressed" tumor antigen or "overexpression" of a tumor antigen is intended to indicate an abnormal level of expression of a tumor antigen in a cell from a disease area like a solid tumor within a specific tissue or organ of the patient relative to the level of expression in a normal cell from that tissue or organ. Patients having solid tumors or a haematological malignancy characterized by overexpression of the tumor antigen can be determined by standard assays known in the art.

The term "administering" refers to contacting, applying, injecting, transfusing or providing a composition of the present invention to a subject.

The term "treating" as used herein may refer to (1) preventing or delaying the appearance of one or more symptoms of the disorder; (2) inhibiting the development of the disorder or one or more symptoms of the disorder; (3) relieving the disorder, i.e., causing regression of the disorder or at least one or more symptoms of the disorder; and/or (4) causing a decrease in the severity of one or more symptoms of the disorder.

The term "synergistic effect" as used herein refers to action of two or three therapeutic agents such as, producing an effect, for example, slowing the progression of a proliferative disease, particularly cancer, or symptoms thereof, which is greater than the simple addition of the effects of each drug administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of a drug combination. The corresponding graphs associated with the equations referred to above are the concentration-effect curve, isobologram curve and combination index curve, respectively.

In particular, the inventors demonstrated in the present invention that combined inhibition of UGT8 and BCL-XL in CRC cell lines provides significant synergy compared to each single agents (see examples herein). Thus, the combinations of the present disclosure provide an effective therapy option capable of improving responses compared to each of the single agents and can lead to more durable responses in the clinic.

The term "UGT8 inhibitor" refers, but is not limited, to an inhibitor of UDP-galactose ceramide galactosyltransferase (UGT8), also known as ceramide galactosyltranferase (CGT;IC50 = 0.2 nM). "UGT8" refers to the protein encoded by the UDP glycosyltransferase 8 gene belonging to the UDP-glycosyltransferase family. It catalyzes the transfer of galactose to ceramide, a key enzymatic step in the biosynthesis of galactocerebrosides, which are abundant sphingolipids of the myelin membrane of the central and peripheral nervous systems. Alternatively spliced transcript variants have been found for this gene. According to the present disclosure, the UGT8 inhibitor may be a compound selected from the group consisting of, UGT8i19 (UGT8 inhibitor 19), RA 5557,or Manidipine-2HCl. In some embodiments, the UGT8 inhibitor may be selected from a list of inhibitors as described in Thurairatnam, Sukanthini et al. "Brain Penetrable Inhibitors of Ceramide Galactosyltransferase for the Treatment of Lysosomal Storage Disorders." ACS medicinal chemistry letters vol. 11,10 2010-2016. 16 Jun. 2020, doi:10.1021/acsmedchemlett.0c00120.

UGT8 inhibitor 19 with formula N-[(1S)-1-methyl-2-(trifluoromethoxy)ethyl]-carbamic acid, 1-[3-[(methylamino)carbonyl]-7-(trifluoromethyl)thieno[3,2-b]pyridin-5-yl]-4-piperidinyl ester is an inhibitor of UDP-galactose ceramide galactosyltransferase (UGT8), also known as ceramide galactosyltranferase (CGT; IC₅₀ = 0.2 nM). It is selective for UGT8 over UGT1A1, -1A6, -2B7, - 2B15, and -2B17 (IC₅₀s = >10 µM for all). *In vivo*, UGT8 inhibitor 19 inhibits the synthesis of galactosylceramide (GalCer) and sulfatide (SFT) in juvenile mouse brain (ED₅₀s = <3 mg/kg for both) and adult mouse kidney when administered at doses of 3, 10, and 30 mg/kg.

In one embodiment, the inhibitor of the UGT8 inhibitor is formulated at a concentration of between 1 nM and 1 M. For example, the pharmaceutical formulation may comprise a UGT8 inhibitor at a concentration of between 1 uM and 1 mM, for example between 1 uM and 100 pM, between 5 uM and 50 uM, between 10 uM and 50 pM, between 20 uM and 40 uM or about 30 uM.

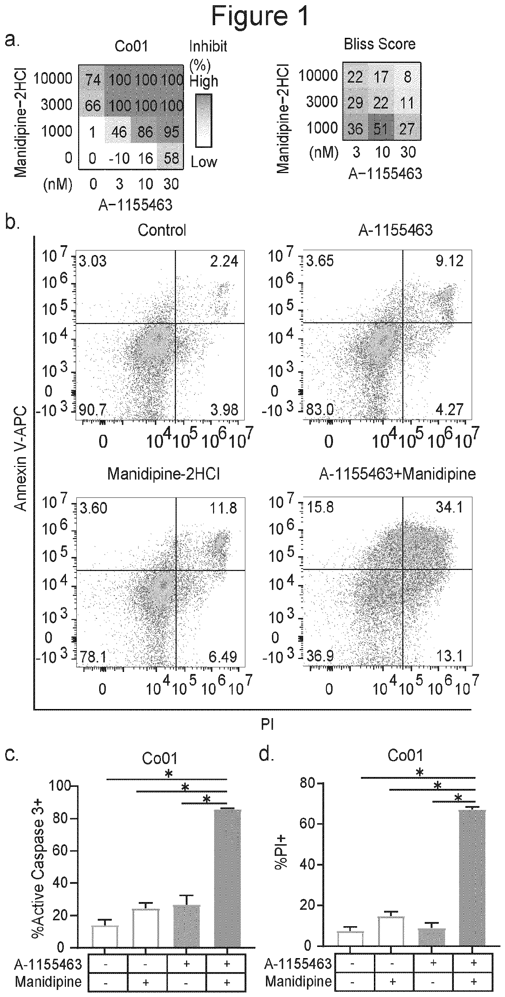

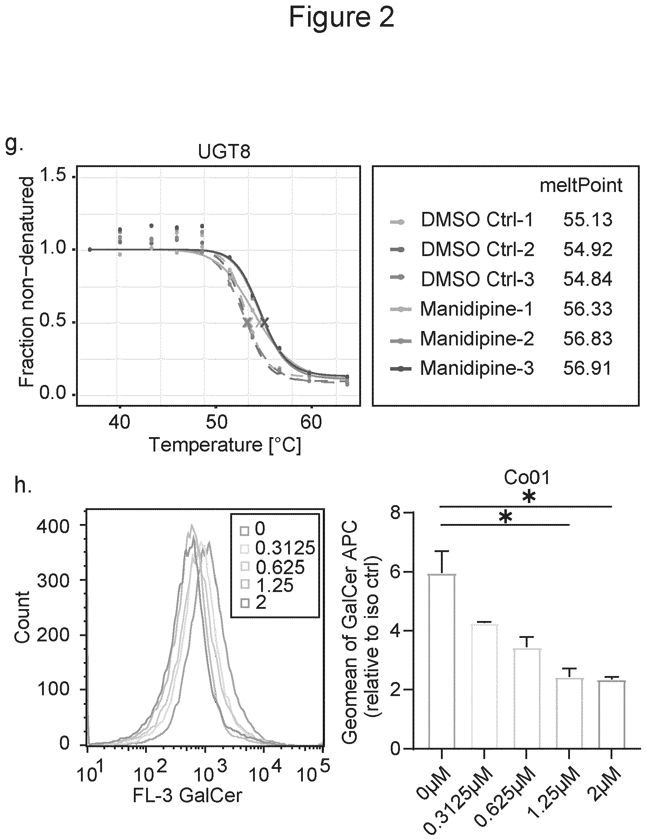

Manidipine-2HCl or Manidipine dihydrochloride with formula 5-O-[2-(4-benzhydrylpiperazin-1-yl)ethyl] 3-*O*-methyl 2,6-dimethyl-4-(3-nitrophenyl)-1,4-dihydropyridine-3,5-dicarboxylate;dihydrochloride is a diarylmethane developed and clinically approved as a calcium channel blocker and anti-hypertensive. In the present disclosure, the inventors demonstrated an unexpected significant and strong reduction in sulfated sphingolipids, specifically the sulfatides (Fig.2g) after treatment of CRC cell lines with Manidipine-2HCl, which demonstrated that Manidipine-2HCl affected sulfatide biosynthesis. The inventors also demonstrated a reduction in GalCer (Figure 2j) which is linked to UGT8 inhibition and to the reduction of the downstream metabolite sulfatide. The inventors showed a surprisingly effective synergy when in combination with low doses of the BCL-XL specific inhibitor A-1155463 in a drug screen (Figure 1a).

The term "Bcl-xL inhibitor" or "BCL-XL inhibitor" or "Bcl-X_{L}inhibitor" is defined herein to refer to a compound which targets, decreases or inhibits the protein Bcl- XL of the anti-apoptotic B-cell lymphoma-2 (Bcl-2) family which is composed of proteins such as Bcl-2, Bcl-X_{L}, Bcl-w, Mcl-1, Bfll/A-1, and/or Bcl-B. The term "Bcl-xL inhibitor" is preferably used herein to refer to those inhibitors which selectively inhibit Bcl-xL and do not inhibit e.g. Bcl-2.

In one embodiment, pharmaceutical combinations of the present disclosure include at least one BCL-XL inhibitor (Tse C, Shoemaker AR, Adickes J, Anderson MG, Chen J, Jin S, et al), preferably that one BCL-XL inhibitor belongs to the groups of BCL-XL inhibitors such as small molecules: A-1155463, A-1331852, WEHI-539, ABT-199 (Venetoclax), ABT-263 (Navitoclax), ABT-737, TW-37, (R)-(-)-Gossypol (AT-101) acetic acid, Sabutoclax, BH3I-1, Gambogic Acid, WL-276, Obatoclax, A-793844, A-1293102; oligomers: p-terphenyl derivative (BH3-M6), oligoamide-foldamer (JY-1-106); naturally derived preferential or dual BCL-XL/Bcl-2/Mcl-1 inhibitors: Anacardic acids, Endiandric acids, Marinopyrroles, Polyphenols (Gossypol, ApoG2, EGCG, TW-37), Meiogynins, and antimycin-A. More preferably the BCL-XL inhibitor is A-1155463.

In one embodiment, the BCL-XL inhibitors may have been modified to have less off-target platelet effects. These modifications may include E3 ligase ligand-based BCL-XL PROTACs (i.e. CRBN: XZ424, XZ739; VHL: DT2216, PZ703b, 753b), prodrug-based approaches, such as antibody-drug conjugates (i.e. ABBV-155), phosphate prodrugs (i.e. APG-1252), dendrimer conjugate (i.e. AZD0466), and glycosylated conjugates (i.e. Nav-Gal).

The term "subject" as used throughout the specification is to be understood to mean a human or may be a domestic or companion animal. While it is particularly contemplated that the methods of the invention are for treatment of humans, they are also applicable to veterinary treatments, including treatment of companion animals such as dogs and cats, and domestic animals such as horses, cattle and sheep, or zoo animals such as primates, felids, canids, bovids, and ungulates. The "subject" may include a person, a patient or individual, and may be of any age or gender.

The term "a therapeutically effective amount" of a compound (e.g. chemical entity or biologic agent) of the present disclosure refers to an amount of the compound of the present disclosure that will elicit the biological or medical response of a subject, for example, reduction or inhibition of an enzyme or a protein activity, or ameliorate symptoms, alleviate conditions, slow or delay disease progression, or prevent a disease, etc. In one embodiment a therapeutically effective amount in vivo may range depending on the route of administration, between about 0.1-500 mg/kg, or between about 1-100 mg/kg.

The optimal dosage of each combination partner for treatment of a cancer can be determined empirically for each individual using known methods and will depend upon a variety of factors, including, though not limited to, the degree of advancement of the disease; the age, body weight, general health, gender and diet of the individual; the time and route of administration; and other medications the individual is taking. Optimal dosages may be established using routine testing and procedures that are well known in the art. The amount of each combination partner that may be combined with the carrier materials to produce a single dosage form will vary depending upon the individual treated and the particular mode of administration. In some embodiments the unit dosage forms containing the combination of agents as described herein will contain the amounts of each agent of the combination that are typically administered when the agents are administered alone. Frequency of dosage may vary depending on the compound used and the particular condition to be treated or prevented. In general, the use of the minimum dosage that is sufficient to provide effective therapy is preferred. Patients may generally be monitored for therapeutic effectiveness using assays suitable for the condition being treated or prevented, which will be familiar to those of ordinary skill in the art.

An effective dose of the Bcl-XL inhibitor can be based in preclinical studies in mice (PROUS integrity records). The dose escalation study in man will allow to identify the maximum tolerated dose, and will allow to define the recommended clinical dose for pivotal clinical studies.

An effective dose of the Bcl-2 inhibitor navitoclax may range from about 100 mg to about 500 mg daily. The dose may be reduced or a 150 mg 7-day lead-in dose employed. After the lead-in dose a 325 mg dose or up to 425 mg dose can be administered daily.

### Detailed description

BCL-XL is a crucial player that support the survival during the progression of colorectal cancer (CRC). Targeting BCL-XL has shown potential in killing CRC cells, but unfortunately also in killing platelets. Therefore, the inventors set up a screening assay to find a sensitizer of BCL-XL inhibitor and investigate the possible pathways involved.

Elevated *de novo* lipid synthesis is a remarkable adaption of cancer cells that can be exploited for therapy. However, the role of altered lipid metabolism in the regulation of apoptosis is still poorly understood. Using thermal proteome profiling, the inventors unexpectedly identified Manidipine-2HCl as a selective inhibitor of UGT8, a key enzyme in the synthesis of sulfatides. In agreement, lipidomic analysis indicated that sulfatides are strongly reduced in colorectal cancer cells upon treatment with Manidipine-2HCl. Intriguingly, this reduction led to severe mitochondrial swelling and a strong synergism with BH3 mimetics targeting BCL-XL, leading to the activation of mitochondria-dependent apoptosis. Mechanistically, Manidipine-2HCl enhanced mitochondrial BAX localization in a sulfatide-dependent fashion, facilitating its activation by BH3 mimetics. This invention presents data that indicate that UGT8 mediated synthesis of sulfatides controls mitochondrial homeostasis and BAX localization, dictating apoptosis sensitivity of colorectal cancer cells.

The inventors demonstrate herein the synergism elicited by Manidipine-2HCl with BH3 mimetic A-1155463 uncovering a completely novel regulatory mechanism that is dependent on sulfatides and organizes mitochondrial morphology as well as regulates BAX localization to the mitochondria. Reduced levels of sulfatides effectively enhance sensitivity to pro-apoptotic insults.

Manidipine-2HCl, a 4^{th} generation dihydropyridine calcium channel antagonist (CCA), was firstly introduced 35 years ago for hypertension treatment with a long lasting calcium channel blocking effect and widely reported to display metabolic benefit in hypertensive patients. However, the present disclosure revealed an off-target effect of Manidipine-2HCl to sensitize CRC cell to apoptosis. In order to pinpoint the direct binder of Manidipine-2HCl responsible for sensitization, the inventors took advantage of thermal proteome profiling (TPP). In principle, TPP can detect target proteins if these change thermostability upon Manidipine binding. However, as thermostability of proteins is determined by several factors, such as dynamic post translational modifications (phosphorylation, ubiquitination) or binding to other proteins, it is difficult to conclude using TPP that Manidipine and UGT8 interact directly. Similarly, it is difficult to determine whether the other hits that were revealed in the TPP analysis represent downstream modified proteins or direct targets of Manidipine-2HCl. Even at such short incubation times (1 hr), the thermostability shift of the proteins identified could be the result of an indirect effect for instance mediated by a change in lipid composition of the cells. The fact that a specific UGT8 inhibitor mirrored the effects observed with Manidipine-2HCl at least confirms that the inhibition of this enzyme is key in the sensitization of CRC cells for the subsequent targeting of BCL-XL. In addition, the reduced Gal-Cer and sulfatide synthesis that was observed with both UGT8-inhibitor and Manidipine-2HCl indicated that the enzymatic activity of UGT8 was affected by Manidipine-2HCl treatment.

UDP-glycosyltransferases (UGTs) are a superfamily of enzymes that catalyze the covalent addition of sugars from nucleotide UDP-sugar donors to a broad range of lipophilic molecules. In mammals, the superfamily comprises four families: UGT1, UGT2, UGT3, and UGT8, which are distinguishably characterized by utilizing distinct UDP-conjugated sugar donors. UGT8, also known as ceramide galactosyltransferase (CGT), uses exclusively UDP-galactose to add galactose to ceramides. Emerging evidence indicates an elevation of this enzyme in different cancers. However, their exact role in the formation of cancer is not fully understood. Under physiological circumstances, UGT8 is crucial for the generation of sulfatides that form an important constituent of the myelin sheath of neurons. More recently, it was reported to be a crucial enzyme in the detoxification of bile acids in the gut, explaining the expression of UGT8 in intestinal epithelium. When looking at the lipid metabolism directly, it is evident that the activity of UGT8 governs the balance between ceramide and its glycosylated metabolites, galactosyl-ceramides (GalCer) and the derived sulfated molecules 3-O-sulfogalactosylceramide, better known as sulfatide. Previously, ceramides were suggested to enhance apoptosis, while other sphingolipids, i.e. sphingosine-1-phosphate and sphingomyelin, which can be generated directly from ceramide, rather protect from cell death. Although controversial, ceramides were suggested to modulate MOMP at early stages of apoptosis and it was suggested that cancer cells detoxify ceramides by glycosylation into either glucosyl-ceramides (GluCer) or galactosyl-ceramides (GalCer). The latter pathway relates to the observed upregulation of UGT8. In agreement, sulfatides are found to be elevated in some cancer cells. Here the inventors provide evidence that sulfatides, contrary to what has been shown in the art, have an anti-apoptotic function, providing an additional rationale for cancer cells to elevate the expression or activity of UGT8, favoring cancer cell survival. Whether UGT8 simply maintains a pro-survival balance in the mitochondria between ceramides and sulfatides remains to be established, but is an interesting option, especially as a clear decrease in the level of sulfatide was also observed in mitochondrial membranes.

The observed sensitization is strongly tumor-selective as no toxicity of the combination treatment was observed in normal colonic organoids even though these cells express UGT8. The inventors' previous work showed that normal colonocytes resist BCL-XL targeting due to the expression of BCL-2, which may explain the lack of toxicity of the combination treatment as well. How sulfatides fit into the regulation of cytochrome C from the mitochondrial membrane remains to be established, but it is clear as demonstrated by the inventors' examples that their synthesis provides a means to regulate mitochondrial homeostasis in CRC. Inactivation of this pathway by Manidipine-2HCl has a major consequence for the shape and function of the mitochondria, allowing these to be covered with BAX that is ready to be activated. Significantly lower amounts of the BCL-XL inhibitor A-1155643 are then sufficient to release the apoptotic machinery. Thus, the current invention discloses an innovative and novel approach of targeting UGT8 in combination with BCL-XL inhibition to treat CRC.

### Figure legends

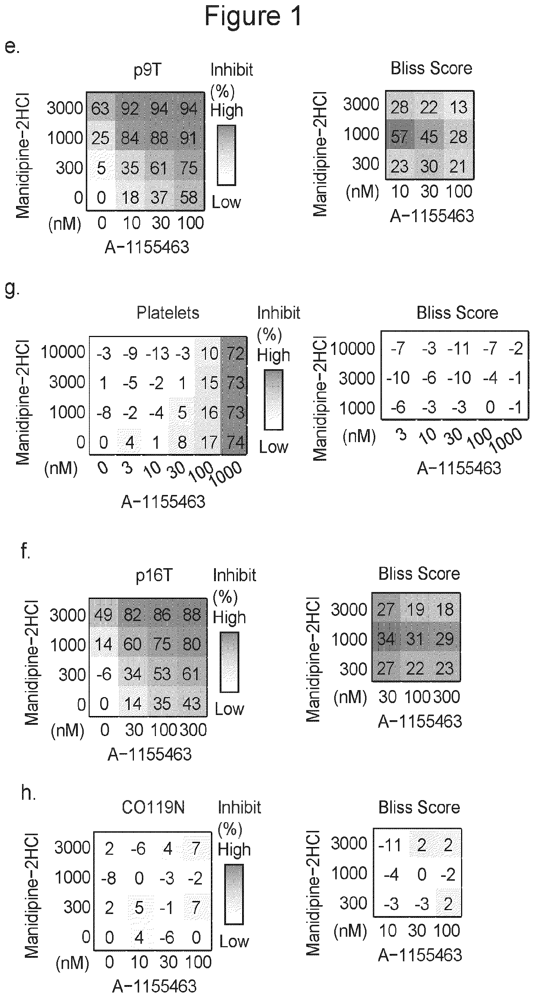

**Fig. 1****. Manidipine-2HCl sensitizes colorectal cancer cells to BCL-XL inhibition.**
   a) Matrix titration and corresponding Bliss synergy scores of the Manidipine-2HCl and A-1155463 in Co01. Cell viability was measured by CellTiter Blue (n=4). Relative inhibition on viability was plotted and Bliss synergy scores were calculated; b) Representative plots of Annexin V and PI FACS upon treatment with or without 2µM Manidipine-2HCl and/or 5nM A-1155463 after 48hrs, determined by flow cytometry. n=4; c)&d) Percentage of Co01 cells showing caspase-3 (c) or PI positivity (d) activity upon treatment with or without 2µM Manidipine-2HCl and/or 5nM A-1155463 after 48hrs, determined by flow cytometry. n=4; e)&f) Matrix titration and corresponding Bliss synergy scores of Manidipine-2HCl and A-1155463 treatment of patient-derived tumor organoids p9T(e) and p16T (f). Cell viability was measured by CellTiter Blue (n=4). Relative inhibition on viability was plotted and Bliss synergy scores were calculated; g) Matrix titration and corresponding Bliss synergy scores of Manidipine-2HCl and A-1155463 treatment in healthy human platelets.1µM A-1155463 was used as positive control for platelet killing. Viability was determined by CellTiter Blue (n=3). Relative inhibition on viability was plotted and the Bliss synergy scores were calculated. h) Matrix titration and corresponding Bliss synergy scores of Manidipine-2HCl and A-1155463 treatment in human normal colon organoids. Viability was determined by CellTiter Glow (n=3). Relative inhibition on viability was plotted and Bliss synergy scores were calculated. Significance was calculated with Mann-Whitney test; ns: not significant. *:p < 0.05.

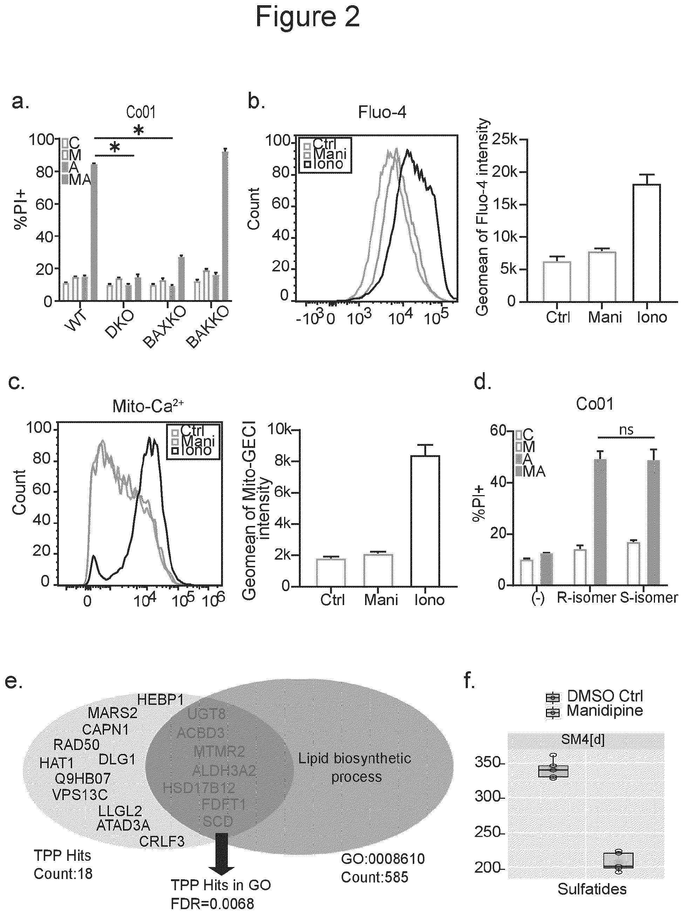

**Fig. 2****. Manidipine-2HCl sensitizes colorectal cancer in a BAX-dependent, but Cav1.1 independent manner and targets UGT8.**
   a) Cell death induced in BAX (BAX-KO), BAK (BAK-KO) single or double (DKO) knock-out Co01 cells treated with or without 2µM Manidipine-2HCl and/or 5nM A-1155463. C: Control; M: Manidipine-2HCl 2µM; A: A-1155463 5nM; MA: Manidipine-2HCl 2µM+A-1155463 5nM. Percentage of PI positive Co01 were determined by flow cytometry and plotted; n=4; b) Intracellular Ca²⁺ levels in Co01 were measured by 1µM Fluo-4. Cells were treated with 2µM Manidipine-2HCl for 24hrs or 10µM Ionomycin for 5mins before measuring by flow cytometry. Geometric means of the fluorescence of fluo-4 was plotted; n=4; c) Mitochondrial Ca²⁺ level in Co01 was measured in cells that contained a transduced mitochondrial targeting Ca²⁺ sensor. Cells were treated with 2µM Manidipine-2HCl for 24hrs or 10µM Ionomycin for 5mins before measuring by flow cytometry. Geometric means of the fluorescence of the mito-Ca²⁺ sensor was plotted; n=4; d) Cell death induced in Co01 treated with or without 2µM of the two enantiomers of Manidipine (R-; S-) and/or 5nM A-1155463. C: Control; M: Manidipine-2HCl 2µM; A: A-1155463 5nM; MA: Manidipine-2HCl 2µM+A-1155463 5nM. Percentage of PI positive Co01 were determined by flow cytometry and plotted; n=4; e) Network cluster analysis of the hits of TPP was performed by STRING v.12. The false discovery rate(FDR) of the association of the cluster with lipid biosynthetic process (GO:008610) was calculated. f) Sulfatide quantification by lipidomic profiling on Co01 treated with or without 2µM Manidipine-2HCl for 24hrs. The relative mass spectometry readout was normalized to the quantity of total input protein; n=5; g) Thermostability curve of UGT8 in Co01 treated with or without 2µM Manidipine-2HCl. Melt points of each treatment were calculated as described in methods; n=3; h) Flow cytometry of galactosyl-ceramide (GalCer) in Co01 treated without (0µM) or with 0.3125, 0.625, 1.25 and 2µM Manidipine-2HCl for 24hrs. Geometric means of the relative fluorescence of GalCer probe to mouse IgG isotype was plotted; n=4;. Significance was calculated with Mann-Whitney test; ns: not significant, *: p < 0.05,.

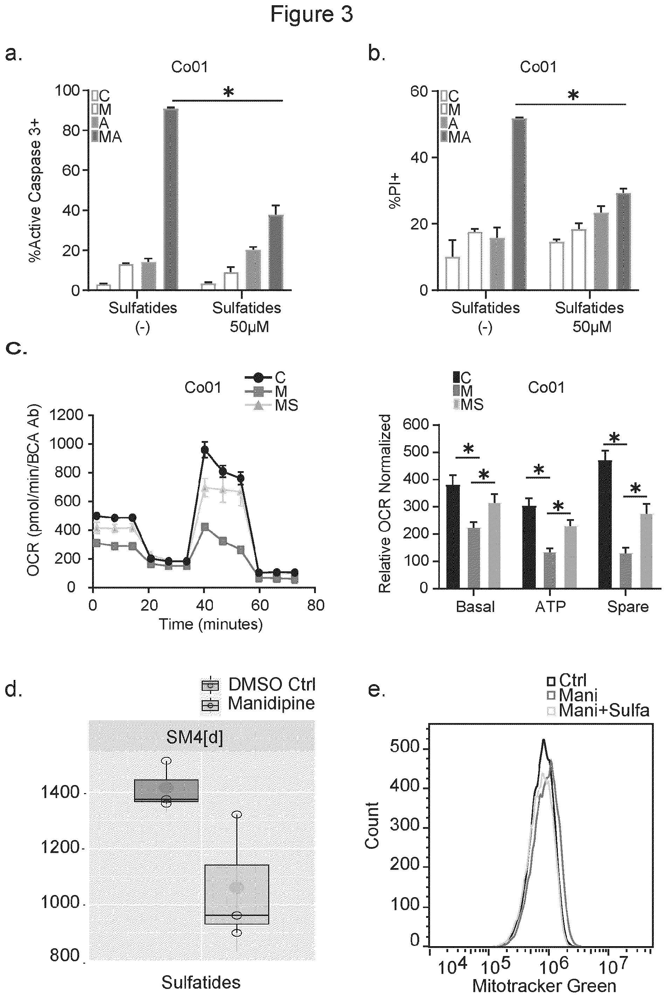

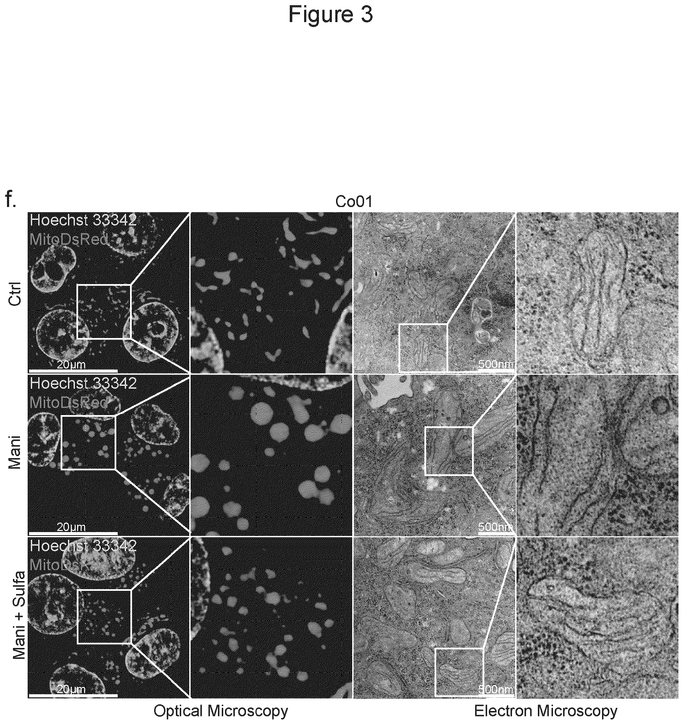

**Fig. 3****. Inhibition of sulfatide synthesis impacted mitochondrial morphology.**
   a) Caspase-3 activity in Co01 cells after treatment for 48hrs with 2µM Manidipine-2HCl and/or 5nM A-1155463 in the presence or absence of 50µM sulfatides as determined by flow cytometry. C: Control; M: Manidipine-2HCl 2µM; A: A-1155463 5nM; MA: Manidipine-2HCl 2µM+A-1155463 5nM; n=4; b) Percentage of PI positive Co01 induced by 2µM Manidipine-2HCl and/or 5nM A-1155463 in the presence or absence of 50µM sulfatides after 48hrs, determined by flow cytometry. C: Control; M: Manidipine-2HCl 2µM; A: A-1155463 5nM; MA: Manidipine-2HCl 2µM+A-1155463 5nM; n=4; c) Co01 treated with or with 2µM Manidipine-2HCl for 24hrs were used to determine the level of sulfatides in isolated mitochondria by lipidomics; n=3. d) Oxygen consumption rate (OCR) of Co01 treated with 2µM Manidipine-2HCl with or without 50µM sulfatides were measured by seahorse mito stress assay. The basal, ATP production related and spare respiratory capacity were plotted as shown by the right lane. C: Control; M: Manidipine-2HCl 2µM; MS: Manidipine-2HCl 2µM+sulfatides 50µM; n=4. e) Mitochondrial mass in Co01 was measured by 50nM Mitotracker Green. Cells were treated with 2µM Manidipine-2HCl with or without 50µM sulfatides for 24hrs before measuring by flow cytometry; n=4. f) The mitochondrial morphology was determined with mitochondria targeting DsRed (MitoDsRed) after treatment with and without 2µM Manidipine-2HCl and/or 50µM sulfatides (left two panels). Mitochondria were imaged with confocal microscope at 63× magnification. Right two panels depict the mitochondrial morphology after fixation and imaging by electron microscopy. Size bars are indicated and significance was calculated with Mann-Whitney test; ns: not significant, *:p < 0.05.

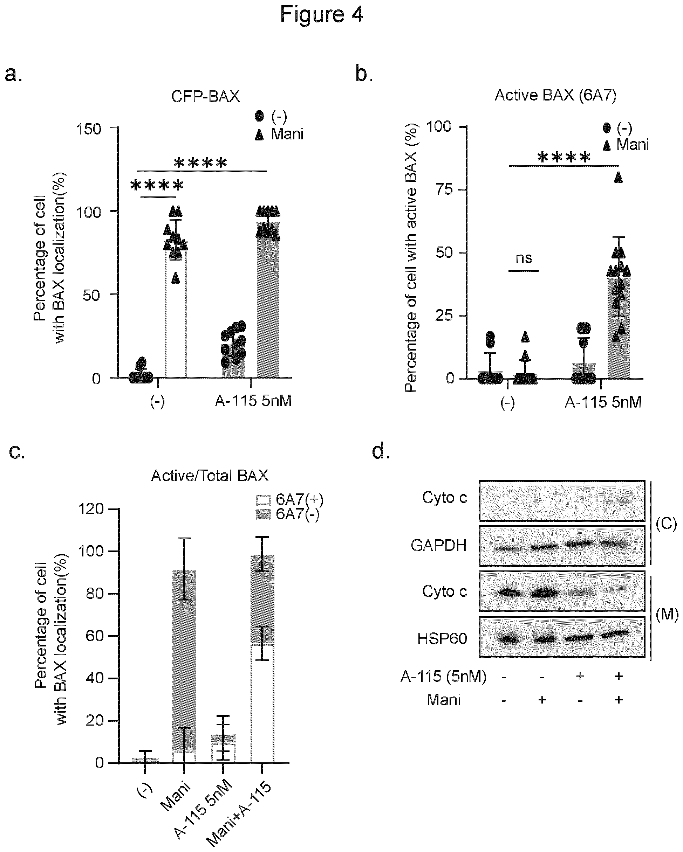

**Fig. 4****. Inhibition of sulfatide regulated BAX localization on the mitochondria.**
   a) CFP-BAX localization was imaged with confocal microscope at 63× magnification. Co01-BAX-KO-CFP-BAX were treated with 2µM Manidipine-2HCl for 24hrs followed by 5nM A-1155463 stimulation for 3hrs. 50nM Mitotracker Deep Red was used to stain mitochondria; CFP-BAX localization was analyzed by using multiple fields of imaging. Percentage of the cells with CFP-BAX to the mitochondria was calculated and plotted; n=10; b) Active BAX was stained with mouse anti-BAX monoclonal antibody 6A7 clone. Co01-BAXKO-CFP-BAX-MitoDsRed were treated with or without 2µM Manidipine-2HCl for 24hrs and where indicated followed by 5nM A-1155463 stimulation for 3hrs before fixation. Images were obtained with confocal microscope at 63× magnification; BAX 6A7 staining were analyzed using multiple fields of imaging. Percentage of the cells with 6A7-positive staining was calculated and plotted; n=16; c) Percentage of cells with active BAX(6A7 positive) in those cells with BAX localization (CFP-BAX localization) to mitochondria. n=6; d) MOMP induction was examined by detecting cytosolic cytochrome c (Cyto c) in Co01 cells treated with 2 Manidipine and 5nM A-1155463 alone or in combination. Both cytosolic(C) and the rest (containing mitochondrial fraction (M)) of cells were blotted. GAPDH and HSP60 were used as the loading control for cytosolic and mitochondrial fraction respectively. Significance was calculated with Mann-Whitney test; ns: not significant, **** p ≤ 0.0001.

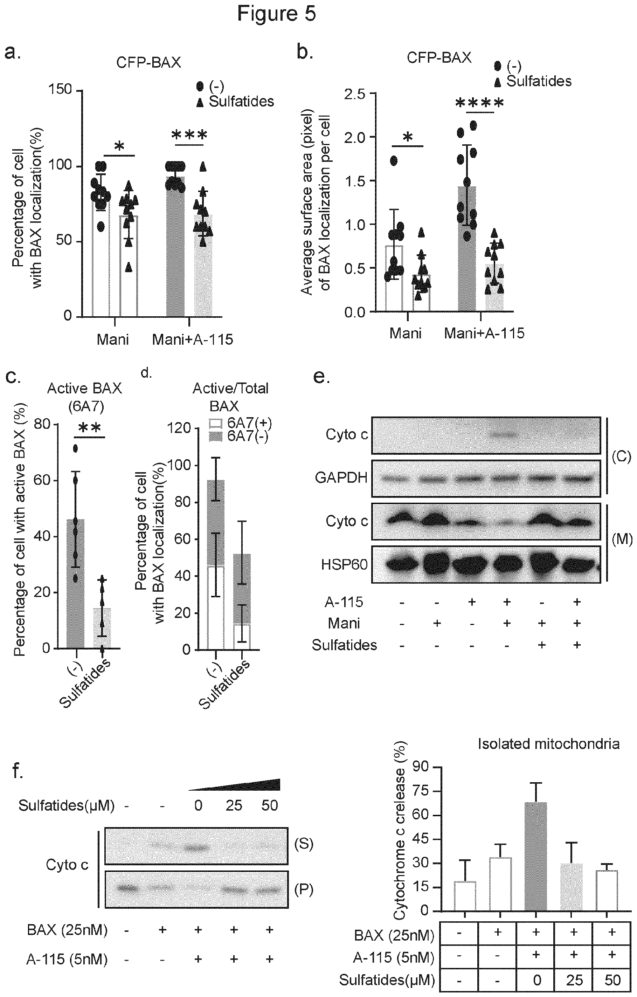

**Fig. 5****. Mitochondrial sulfatide regulated BAX localization and directly prevented MOMP.**
   a) Quantification of the cells positive for CFP-BAX localization to the mitochondria; CFP-BAX localization was imaged with confocal microscope at 63× magnification. Co01-BAX-KO-CFP-BAX were treated with 2µM Manidipine-2HCl with or without 50µM sulfatides for 24hrs followed by 5nM A-1155463 stimulation for 3hrs. 50nM Mitotracker Deep Red was used to stain mitochondria; n=10; and b) the average surface areas of the fluorescent spots per cell treated with 2µM Manidipine-2HCl with or without 50µM sulfatides for 24hrs followed by 5nM A-1155463; n=10; c) Active BAX immunofluorescent staining with mouse anti-BAX monoclonal antibody 6A7 clone. Co01-BAXKO-CFP-BAX-MitoDsRed were treated with 2µM Manidipine-2HCl with or without 50µM sulfatides for 24hrs and where indicated followed by 5nM A-1155463 stimulation for 3hrs before fixation. Images were obtained with confocal microscope at 63× magnification; BAX 6A7 staining were analyzed using multiple fields of imaging. Percentage of the cells with 6A7-positive staining was calculated and plotted; n=6; d) Percentage of cells with active BAX(6A7 positive) in those cells with BAX localization (CFP-BAX localization) to mitochondria, n=6; e) MOMP induction was examined by detecting cytosolic cytochrome c (Cyto c) in Co01 cells treated with 2 Manidipine and 5nM A-1155463 alone or in combination in the presence or absence of 50µM sulfatides. Both cytosolic(C) and the rest (containing mitochondrial fraction (M)) of cells were blotted. GAPDH and HSP60 were used as the loading control for cytosolic and mitochondrial fraction respectively. f) In vitro cytochrome c release assay was performed on isolated mitochondria pretreated with or with sulfatides. Both the released fraction (S) and the pellet fraction (P) of mitochondria were blotted. Grayscale of bands was quantified by imaged and relative release of cytochrome c (supernatant: pellet) was calculated and plotted, n=3. Significance was calculated with Mann-Whitney test; ns: not significant, *:p < 0.05, **: p ≤ 0.01, ***: p ≤ 0.001, **** p ≤ 0.0001.

### Examples

### Example 1. Manidipine-2HCl sensitizes colorectal cancer cells to BCL-XL inhibition

To find a safe and CRC-selective combination therapy, the inventors utilized low doses of the BCL-XL specific inhibitor A-1155463 in combination with Manidipine-2HCl, which the inventors have previously shown to provide effective synergy in a drug screen (Fig.1a). Manidipine-2HCl, which is developed and clinical approved as calcium channel blocker and anti-hypertensive, displayed the highest synergy as calculated with Bliss(28). To validate the effectiveness of the combination treatment in a broader set of CRC lines and over a broader concentration range, both drugs were titrated alone or in combination on 3 independent primary patient-derived CRC lines (Co01, Co108, Co147). In all cases very effective synergy was detected (Fig.1b, Extended Data Fig.1a,b). Importantly, the drop in cell viability as measured by the CellTiter Blue assay was the result of cell death achieved by the combination treatment as indicated by massive induction of caspase-3 activity as well as propidium iodide (PI) influx into the cells (Fig.1c,d and Extended Data Fig.1c,d). Next to the synergistic toxicity on primary spheroid lines, the synergy was studied in two patient-derived tumor organoids (Fig.1e,f) and in a panel of 11 CRC cell lines (Extended Data Fig.1e-o). Although the concentration at which synergy was detected was higher in the CRC cell lines, a result of the use of fetal calf serum in these cultures, in all cases the combination resulted in a strong induction of cell death. Notably, in contrast to all CRC cell lines and primary cultures tested, both platelets (Fig.1g) and normal colon epithelial organoid cultures (Fig.1h) resisted the combination of Manidipine-2HCl and A-1155463, which showed that the toxicity of the combination treatment is tumor selective.

### Example 2. Manidipine-2HCl sensitizes colorectal cancer in a BAX-dependent, but Cav1.1 independent manner

BAX and BAK are crucial mediators of mitochondrial-dependent apoptosis and therefore expected to coordinate A-1155463-dependent apoptosis. To determine whether these canonical executors of apoptosis are also crucial for the synergism, BAX and BAK single (KO) and double knockouts (DKO) CRC lines were generated. Intriguingly, whereas BAK-KO cells were equally sensitive to the combination treatment, both the BAX-KO and the BAX/BAK-DKO lines were completely resistant to Manidipine-2HCl and A-1155463 treatment (Fig.2a), showing that BAX is the key regulator of cell death induction in this combination treatment.

Manidipine-2HCl is reported to target L type Ca²⁺ channel subunit α1 (Cav1.1) and prevent calcium fluxes over the plasma membrane. However, mRNA expression of Cav1.1 revealed a variable and often very low expression in the CRC lines that showed sensitivity to the combination treatment. Moreover, the cytosolic Ca²⁺ levels, as assessed by the Ca²⁺ fluorescent probe Fluo-4, rather increased than decreased after a 24hr Manidipine-2HCl treatment (Fig.2b). Importantly, mitochondrial Ca²⁺ levels were not affected by Manidipine-2HCl (Fig.2c), showing that Manidipine-2HCl may not sensitize CRC lines through inhibition of L type Ca²⁺ channels. In agreement, the two enantiomers of Manidipine-2HCl both showed similar activity in sensitizing CRC cells to A-1155463 (Fig.2d), while the S-Manidipine-2HCl form has a reported 30-80 times higher potency in inhibiting Cav1.1(29). Similarly, four other dihydropyridines, all effective Cav1.1 inhibitors, did not synergize with 5nM A-1155463 (Extended Data Fig.2a-d). Combined these observations indicated that Manidipine-2HCl displayed off-target activity in its sensitization of CRC cells to the BCL-XL inhibitor.

### Example 3. Inhibition of UGT8-induced sulfatide formation is central to apoptosis induction

To identify the target of Manidipine-2HCl, the inventors made use of thermal proteome profiling (TPP). As illustrated Extended Data Fig. 2e, binding of Manidipine-2HCl to a target is expected to alter the biochemical properties of the target resulting in a potential melting temperature shift. To maximize the potential to identify direct binders of Manidipine-2HCl, the inventors performed the TPP analysis 1 hour after the addition of Manidipine-2HCl to the CRC cells and found that 18 proteins significantly shifted their melting curves (Extended Data Fig.2f). Strikingly, 7 out of these 18 proteins were associated with lipid biosynthesis (Fig.2f). Therefore, to determine whether Manidipine-2HCl impacted the lipid composition of cells, lipidomics was performed. Interestingly, the majority of lipids did not reveal significant changes upon treatment. However, a significant and strong reduction was observed in sulfated sphingolipids, specifically the sulfatides (Fig.2g), which demonstrated that Manidipine-2HCl affected sulfatide biosynthesis (Fig.2h). This is in line with the TPP analysis in which UGT8 displayed the strongest shift in its melting curve (Fig.2i and Extended Data Fig.2f). Unfortunately, the direct product of the reaction catalyzed by UTG8, galactosyl-ceramide (GalCer) cannot be directly monitored by mass spectometry analysis as it is identical to its diastereoisomer glucosyl-ceramide, which is part of another biosynthethic pathway. To circumvent this, an antibody to GalCer was used and staining of Manidipine-2HCl-treated cells confirmed the significant reduction in GalCer (Fig.2j), which subsequently leads to the reduction of the downstream metabolite sulfatide. To confirm the role of the sulfatide biosynthesis in sensitization of CRC cells to the BCL-XL inhibitor A-1 155463, a selective UGT8 inhibitor, UGT8i19, was tested. Compared to Manidipine-2HCl, UGT8i19was even more potent in decreasing sulfatides and GalCer levels in CRC cells (Extended Data Fig.2g). Intriguingly, UGT8i19 also sensitized CRC cells to low dose of the BCL-XL inhibitor (Extended Data Fig.2i), further supporting the hypothesis that the lipid metabolism pathway controlled by UGT8 is crucial in defining the impact of BH3 mimetics targeting BCL-XL.

### Example 4. Inhibition of sulfatide synthesis impacted on mitochondrial morphology

UGT8 is often upregulated in cancer and its expression is associated with poor prognosis in breast cancer patients(30). As inhibition of this enzyme by UGT8i19 or Manidipine-2HCl had a major impact on the cellular levels of sulfatide, exogenously added sulfatides should be able to revert the synergistic induction of cell death. In agreement, addition of 50µM of sulfatides to the culture medium could significantly rescue multiple CRC lines from Manidipine-2HCl/A-1155463-induced apoptotic cell death (Fig.3a,b and Extended Data Fig.3a,b). As expected, exogenously added sulfatides also rescued CRC cells from the combination treatment of UGT8i19 and A-1155463 (Extended Data Fig.3c). Intriguingly, sulfatides were also detected by mass spectrometry lipid analysis in the mitochondrial fraction of CRC cells and displayed a significant reduction in quantity upon Manidipine-2HCl treatment alone (Fig.3c), suggesting that mitochondrial homeostasis could depend on sulfatides. In agreement, Manidipine-2HCl induced massive changes in the mitochondrial morphology of CRC cells. Firstly, mitochondrial respiration was strongly impaired by treatment with Manidipine-2HCl in multiple CRC cell lines and this was partly restored by sulfatides (Fig.3d and extended data Fig.3d,e). This reduction is not due to a change in the mitochondrial content as quantification of mitochondrial mass with Mitotracker Green did not reveal differences (Fig.3e). Subsequent visualization of the mitochondria with mito-DsRed indicated that Manidipine-2HCl treated cells contained heavily swollen mitochondria (Fig.3f). This was confirmed by electron microscopy, which pointed to an inflated mitochondrial matrix (Fig.3f). The role of sulfatide, and specifically the reduction in the levels of sulfatide, in these morphological changes was again confirmed by the addition of exogenous sulfatides (Fig.3f), demonstrating that they play a crucial role in mitochondrial function.

### Example 5. Mitochondrial sulfatide regulated BAX localization on the mitochondria.

The central role of BAX in the combination treatment, which mediates mitochondrial outer membrane permeabilization (MOMP), combined with the impact on mitochondrial morphology induced by the sulfatide reduction, prompted us to analyze BAX localization dynamics. To this end, CFP-BAX was transduced in Co01-BAX-KO cells, which resulted in relatively high BAX levels, but these did neither induce apoptosis by themselves nor sensitize the cells to BCL-XL inhibition (Extended Data Fig.4a). Under normal conditions BAX was dispersed in the cytoplasm and only little BAX was found to be localized to the mitochondria. In contrast, 24 hours of Manidipine-2HCl treatment alone resulted in a significant shift in BAX localization, with close to a 100% of the cells displaying mitochondria with BAX aggregates (Extended Data Fig.4c). A similar shift in BAX localization was induced by UGT8i19 (Extended Data Fig.4c). Surprisingly, the extend of BAX localization to the mitochondria could be further enhanced by addition of low dose of A-1155463. Although, BAX localization to the mitochondrial membrane is a prerequisite for MOMP, it is by itself not sufficient to permeabilize the mitochondrial membrane as BAX requires further activation to insert into the lipid bilayer and form pore forming structures. Interestingly, although Manidipine-2HCl induced the mitochondrial localization of BAX, it failed to induce the insertion into the membrane as shown by the absence of staining with the 6A7 antibody that recognizes the active/inserted conformation (Fig.4a). In contrast, the addition of A-1155463 quickly changed the BAX state from a mitochondrial residing form to an mitochondrial outer membrane-inserted form leading to the rapid induction of MOMP (Fig.4a). Strikingly, exogenously added sulfatides dramatically reduced the Manidipine-2HCl-induced localization of BAX to the mitochondria both in the amount of cells showing localization and in the extend of the localization (Fig.4b,c), providing further evidence that a change in the mitochondrial lipid constitution regulates mitochondrial function as well as mitochondrial BAX localization. Manidipine-2HCl or UGT8i19 treated cells are therefore primed to undergo apoptosis due to an enhanced BAX localization to the mitochondria, ready to unleash MOMP. A simply push by A-1155463 is then sufficient to release the brake and induce MOMP (Fig.4d).

## Claims

1. A combination of a UGT8 inhibitor and a BCL-XL inhibitor for use in a method of treating a patient suffering from cancer.

2. The combination of a UGT8 inhibitor and a BCL-XL inhibitor for use according to claim 1 wherein the UGT8 inhibitor is UGT8 inhibitor is Manidipine-2HCl, RA 5557, or UGT8i19 (UGT8 inhibitor 19). More preferably the UGT8 inhibitor is Manidipine-2HCl.

3. The combination of a UGT8 inhibitor and a BCL-XL inhibitor for use according to any one of the previous claims wherein the BCL-XL inhibitor belongs to the groups of BCL-XL inhibitors such as small molecules: A-1155463, A-1331852, WEHI-539, ABT-199 (Venetoclax), ABT-263 (Navitoclax), ABT-737, TW-37, (R)-(-)-Gossypol (AT-101) acetic acid, Sabutoclax, BH3I-1, Gambogic Acid, WL-276, Obatoclax, A-793844, A-1293102; oligomers: p-terphenyl derivative (BH3-M6), oligoamide-foldamer (JY-1-106); naturally derived preferential or dual BCL-XL/Bcl-2/Mcl-1 inhibitors: Anacardic acids, Endiandric acids, Marinopyrroles, Polyphenols (Gossypol, ApoG2, EGCG, TW-37), Meiogynins, and antimycin-A. More preferably the BCL-XL inhibitor is A-1155463.

4. The method of treatment according to claim 1 wherein the cancer type is colorectal cancer.

5. A pharmaceutical preparation, comprising a UGT8 inhibitor and a BCL-XL inhibitor as a combined preparation for simultaneous, separate or sequential use in the treatment of colorectal cancer in a subject.

6. The pharmaceutical preparation according to embodiment 5, comprising a pharmaceutical preparation comprising a UGT8 inhibitor and a pharmaceutical preparation comprising a BCL-XL inhibitor.

7. The pharmaceutical preparation according to embodiment 5 or embodiment 6, for use in a method of treating colorectal cancer.

8. A method of treating a colorectal cancer in a subject, the method comprising the simultaneous, separate or sequential administration to the subject of a UGT8 inhibitor and a BCL-XL inhibitor or the pharmaceutical preparation from embodiment 5 to the subject.
